# EUROPEAN PATENT APPLICATION

(11) **EP 1 162 551 A2**
(43) Date of publication of application: **12.12.2001**
(21) Application number: 01113852.6
(22) Date of filing: 07.06.2001
(51) Int. Cl.: G06F 17/30

(54) **Method for bi-level customization of programs**

(30) Priority: 09.06.2000 US 590963
(71) Applicant: PeakCare LLC, San Diego, California 92130 (US)
(72) Inventor: Leeds, Gary, Rancho Santa Fe, California 92067 (US); Leeds, Jordan, San Diego, California 82130 (US)
(74) Representative: Walsh, Michael Joseph

(57) **Abstract**

A system and method for establishing a customized visual presentation, for on-site use to foster worksite wellness, requires that a provider compile a plurality of information modules. Each module pertains to an occupational descriptor, such as supervisory, clerical, strenuous or non-strenuous manual labor, custodial, or other, and within each descriptor there is an occupational program with subject areas, such as safety, work hazards, environmental illnesses, worksite injuries, and performance motivation. In operation, an intermediary obtains modules from the provider and presents these modules for selective accessibility at a worksite. An individual end user at the worksite can then customize a selected occupational program with personal data and retrieve digitized excerpts from the provider, through the intermediary over an internet connection. The digitized excerpts are thus presented in conformance with and in accordance with the personal data, the occupation program and the information module to establish the visual presentation at the worksite.

## Description

### FIELD OF THE INVENTION

The present invention pertains generally to methods and systems for presenting information about worksite wellness to an individual end user at his/her worksite. More particularly, the present invention pertains to audio/visual on-site presentations that are accessible by an individual end user over a global computer network, such as the internet. The present invention is particularly, but not exclusively, useful as a method and system for customizing an audio/visual presentation that is specifically directed to the particular concerns and interests related to an individual end user's work environment and occupational specialty.

### BACKGROUND OF THE INVENTION

Worksite wellness is an issue of vital importance to any business concern. As a practical matter, it is equally important to both the employer and the employee. Within the ambit of worksite wellness are such concerns as: morale of the workforce, productivity, quality of products and services, and the overall health of individuals in the workplace. The various aspects of these concerns are far-reaching and include, in part, considerations of mental health, behavioral and psychological issues, as well as physical and medical ailments. Suffice it to say, the subject of worksite wellness is extensive. In the final analysis, however, the bottom line issue concerns the overall well being of the individual worker.

The key to effectively addressing the issues associated with worksite wellness is actually twofold. First, if possible, it is preferable to anticipate a potentially debilitating situation and take the appropriate corrective action before there are any adverse consequences. Hopefully, the appropriate corrective action can be taken by the individual worker without the involvement of others. Second, if necessary, it is desirable that the individual worker's situation be evaluated and properly referred to appropriate professional help as early as possible. In both instances, education of the individual worker is essential.

In order to properly educate a workforce about worksite wellness, there are several considerations which need to be taken into account. Specifically, there are many considerations which involve the individual needs of the particular worker. Additionally, there are considerations which depend on the specific requirements of the occupational tasks that are performed by the particular worker. These include considerations of both the physical and mental requirements imposed on the worker and the environmental conditions of the worksite. Further, there are considerations which affect other workers, such as support and administrative personnel, as well as the owner/employer.

In light of the above it is an object of the present invention to provide a method and system for establishing a customized visual presentation for on-site use to foster worksite wellness which is job-specific for the individual worker. Another object of the present invention is to provide a method and system for establishing a customized visual presentation for on-site use to foster worksite wellness which is instantaneously responsive to the inquiries of an individual worker. Still another object of the present invention is to provide a method and system for establishing a visual presentation for on-site use to foster worksite wellness which can be customized and individually tailored to the needs of a particular end user. Yet another object of the present invention is to provide a method and system for establishing a customized visual presentation for on-site use to foster worksite wellness which is easy to use, simple to access or install and comparatively cost effective.

### SUMMARY OF THE PREFERRED EMBODIMENTS

In accordance with the present invention, a system for establishing a customized visual presentation for on-site use, that is tailored to foster worksite wellness, essentially requires effective communications between a provider (a source for information), an intermediary (owner/employer), and an individual end user (the worker/employee). More specifically, as envisioned for the present invention, communications between the provider and the intermediary are conducted over a global computer network, such as the internet. Internal company communications between the intermediary and the end user, however, are to be conducted over a local communications system, such as wiring between a central console and personal computers at various worksites in the company. Within this framework, communications between the end user and the provider will be conducted over the internet through the intermediary.

As intended for the present invention, communications between the provider and the end user will be facilitated and controlled by the intermediary, and will pertain directly to the end user, and to his/her occupational well being, at his/her worksite. Further, all information pertaining to worksite wellness that is communicated to the end user by the provider, will be in the form of an audio/visual presentation, and can be customized for the particular end user. The presentation can then be subsequently validated and modified, if necessary, by the particular end user.

The communications format for the present invention envisions the creation of various information modules. This is accomplished by the provider. Specifically, each information module will pertain to a particular occupational descriptor that identifies an occupational specialty such as a supervisory descriptor, a clerical descriptor, a strenuous manual labor descriptor, a non-strenuous manual labor descriptor, a custodial descriptor, or some other appropriate descriptor. Further, within each information module there are a plurality of occupational programs. Specifically, each occupational program will pertain to a selected subject area, such as safety, work hazards, environmental illnesses, worksite injuries, or performance motivation. Thus, an intermediary (owner/employer) can select specific information modules for presentation at a particular worksite and, further, the intermediary can select specific occupational programs for inclusion in the information module. For example, an intermediary can arrange with the provider to establish on-site access for an end user to an audio/visual presentation that gives worksite wellness information to clerical workers about environmental illnesses in their particular industry.

It is an important aspect of the present invention that the end user be able to interact with the information module that is accessible at the worksite. Specifically, as intended for the present invention the end user is given the opportunity to provide personal data which can be used to customize the occupational program in the information module. In compliance with this personal data, the system then retrieves digitized excerpts from the provider through the intermediary over an internet connection. Specifically, these excerpts will pertain to the occupation program and the information module that are to be included in the audio/visual presentation at the worksite. Further, the system and method of the present invention envisions the ability of the end user to validate the occupational program at the worksite for the specific needs of the end user, and to modify the occupational program as necessary.

### BRIEF DESCRIPTION OF THE DRAWINGS

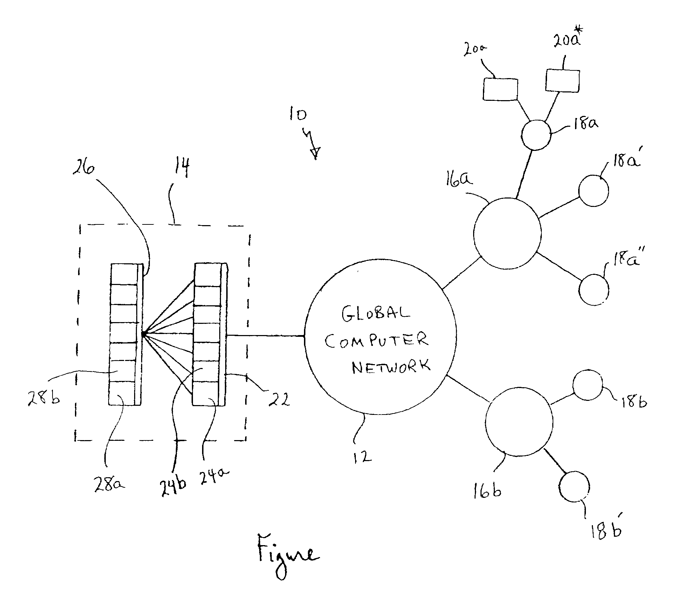

The novel features of this invention, as well as the invention itself, both as to its structure and its operation, will be best understood from the accompanying drawings, taken in conjunction with the accompanying description, in which similar reference characters refer to similar parts, and in which:
The Figure is a schematic presentation of the system of the present invention showing the interrelationship of the parties through their respective communications links with each other.

### DESCRIPTION OF THE PREFERRED EMBODIMENT

Referring to the Figure, a system for establishing a customized audio/visual presentation for on-site use, that is designed to foster worksite wellness in accordance with the present invention, is shown and generally designated 10. As shown, the system 10 involves a communications scheme with links over a global computer network, such as the internet 12. Specifically, these communications links for the system 10 are established between a provider 14, an intermediary 16 and a worksite 18. With reference to the Figure, it is to be appreciated that the intermediaries 16a and 16b are only exemplary, and that many such intermediaries 16 can be included. Specifically, an intermediary 16 will be any owner/employer, organization or group of affiliates that incorporates the system 10 into his/her/their business for the purpose of providing employees with effective information about worksite wellness. For the system 10, the worksites 18a, 18a' and 18a" that are associated with the intermediary 16a, as well as the worksites 18b and 18b' that are associated with the intermediary 16b are only exemplary. As envisioned for the system 10 of the present invention, there may be as many worksites 18 associated with an intermediary 16 as are desired by the particular intermediary 16. Further, each worksite 18 should be as accessible to the intended end user as is possible. Preferably, each worksite 18 will incorporate a personal computer 20 for use by the end user. The particular personal computers 20a and 20a shown associated with the worksite 18a are only exemplary.

The format for communications over the system 10 will, perhaps, be best appreciated with reference to the provider 14. As shown, it is possible to access an information module 22 through the provider 14. Actually, the provider 14 is able to offer several different types of information modules 22. The particular type of information module 22 offered by the provider 14 will depend on the nature of the industry involved and, more specifically, on the occupational descriptor 24 for which the information module 22 was prepared. Typical occupational descriptors 24 will include classifications chosen from a group that can include a supervisory descriptor, a clerical descriptor, a strenuous manual labor descriptor, a non-strenuous manual labor descriptor, a custodial descriptor, and any other appropriate descriptor. Thus, for example, one information module 22 may be specifically prepared with an occupational descriptor 24a for supervisory personnel, while another information module 22 may be specifically prepared with an occupational descriptor 24b for persons engaged in strenuous manual labor activities.

As also shown in the Figure, according to its occupational descriptor 24, each information module 22 can be directed toward an occupational program 26. Further, each occupational program 26 will include digital excerpts for various subject areas 28 such as safety, work hazards, environmental illnesses, worksite injuries, and performance motivation. For example, one occupational program 26 may pertain to a subject area 28a that addresses safety issues while another occupational program 26 will pertain to a subject area 28b that involves performance motivation issues. Again, by way of example, the consequence of the above is that a warehouse employee (end user), whose job involves lifting and moving heavy objects, can use a personal computer 20 at his/her worksite 18, and through the intermediary 16 gain access over the internet 12 to the provider 14. This particular end user could then identify an occupational descriptor 24 (e.g. occupational descriptor 24b) for an information module 22, and then choose an occupational program 26 with safety as its subject area 28 (e.g. subject area 28a). As will be appreciated by the skilled artisan, within the scheme, there are many commutations and permutations to the information modules 22 and occupational programs 26 that are available to the end user over the system 10.

It is an important aspect of the present invention that the intermediary 16 be able to select the particular information modules 22 that are to be made available to end users at worksites 18 operated by the intermediary 16. With this in mind, it is thus possible for the intermediary 16 to tailor the information modules 22 and occupational programs 26 for inclusion in a worksite wellness stratagem that will benefit his/her employees. Of equal importance is the ability of the end user (employee) to customize her/his information module 22 and occupational program 26. Specifically, as envisioned for the system 10 of the present invention, an end user at a personal computer 20, at a worksite 18, is able to provide personal information that will specifically customize the particular occupational program 26 for his/her needs. The resultant audio/visual presentation will then be in conformance with, and in accordance with, the personal data, the occupational program 26, and the selected information module 22. Further, at the worksite 18, the end user will be able to validate the audio/visual presentation and modify it as necessary.

While the particular Method for Bi-Level Customization of Programs as herein shown and disclosed in detail is fully capable of obtaining the objects and providing the advantages herein before stated, it is to be understood that it is merely illustrative of the presently preferred embodiments of the invention and that no limitations are intended to the details of construction or design herein shown other than as described in the appended claims.

## Claims

1. A method for establishing a customized visual presentation for on-site use to foster worksite wellness which comprises the steps of:
selecting an information module for said worksite, said information module pertaining to an occupational descriptor specified by an intermediary;
identifying an occupational program within said information module;
providing personal data from an end user to customize said occupational program; and
retrieving digitized excerpts from a provider through said intermediary over an internet connection, said digitized excerpts being presented in conformance with and in accordance with said personal data, said occupation program and said information module to establish said visual presentation at said worksite.

2. A method as recited in claim 1 wherein said intermediary provides a plurality of information modules for use in said selecting step.

3. A method as recited in claim 2 wherein each said occupational descriptor for a respective said information module is chosen from a group including a supervisory descriptor, a clerical descriptor, a strenuous manual labor descriptor, a non-strenuous manual labor descriptor, a custodial descriptor, and other.

4. A method as recited in claim 1 wherein each said module includes a plurality of occupational programs.

5. A method as recited in claim 4 wherein each said occupational program includes information pertaining to selected subject areas, and wherein said subject areas are chosen from a group including safety, work hazards, environmental illnesses, worksite injuries, and performance motivation.

6. A method as recited in claim 1 further comprising the step of validating said occupational program at said worksite for adaptation by said end user.

7. A method as recited in claim 6 further comprising the step of modifying said providing step in response to said validating step.

8. A method for establishing a customized visual presentation for on-site use to foster worksite wellness which comprises the steps of:
compiling a plurality of information modules, with each said information module pertaining to an occupational descriptor;
combining a plurality of occupational programs within each said information module;
presenting said plurality of information modules for selective accessibility at a worksite;
customizing a selected said occupational program with personal data from an end user at said worksite; and
retrieving digitized excerpts from a provider and through an intermediary over an internet connection for use at said worksite, said digitized excerpts being presented in conformance with and in accordance with said personal data, said occupation program and said information module to establish said visual presentation at said worksite.

9. A method as recited in claim 8 further comprising the steps of:
validating said occupational program at said worksite for adaptation by said end user; and
modifying said providing step in response to said validating step.

10. A method as recited in claim 8 wherein said compiling step and said combining step are accomplished by said provider.

11. A method as recited in claim 8 wherein said presenting step is accomplished by said intermediary.

12. A method as recited in claim 8 wherein said retrieving step is accomplished by an individual at said worksite.

13. A method as recited in claim 8 wherein each said occupational descriptor for a respective said information module is chosen from a group including a supervisory descriptor, a clerical descriptor, a strenuous manual labor descriptor, a non-strenuous manual labor descriptor, a custodial descriptor, and other.

14. A method as recited in claim 8 wherein each said occupational program includes information pertaining to selected subject areas, and wherein said subject areas are chosen from a group including safety, work hazards, environmental illnesses, worksite injuries, and performance motivation.

15. A system for establishing a customized visual presentation for on-site use to foster worksite wellness which comprises:
a provider for compiling a plurality of information modules, with each said information module pertaining to an occupational descriptor, and for combining a plurality of occupational programs within each said information module;
a means for connecting said provider in communication with an intermediary via an internet connection;
a means for allowing said intermediary to present said plurality of information modules for selective accessibility at a worksite; and
a means for customizing a selected said occupational program with personal data from an end user at said worksite, and for retrieving digitized excerpts from said provider through said intermediary over said internet connection for use at said worksite, said digitized excerpts being presented in conformance with and in accordance with said personal data, said occupation program and said information module to establish said visual presentation at said worksite.

16. A system as recited in claim 15 further comprising:
a means for validating said occupational program at said worksite for adaptation by said end user; and
a means for modifying said providing step in response to said validating step.

17. A system as recited in claim 15 wherein each said occupational descriptor for a respective said information module is chosen from a group including a supervisory descriptor, a clerical descriptor, a strenuous manual labor descriptor, a non-strenuous manual labor descriptor, a custodial descriptor, and other.

18. A system as recited in claim 15 wherein each said occupational program includes information pertaining to selected subject areas, and wherein said subject areas are chosen from a group including safety, work hazards, environmental illnesses, worksite injuries, and performance motivation.

19. A system as recited in claim 15 wherein said means for customizing a selected said occupational program and for retrieving digitized excerpts from said provider through said intermediary over said internet connection is a personal computer.
